# EUROPEAN PATENT APPLICATION

(11) **EP 4 740 990 A1**
(43) Date of publication of application: **13.05.2026**
(21) Application number: 24212307.3
(22) Date of filing: 12.11.2024
(51) Int. Cl.: A61M 25/01, A61B 5/00, A61B 34/00, A61B 18/00, G06F 3/01

(54) **SYSTEM AND HAPTIC FEEDBACK DEVICE TO PROVIDE HAPTIC FEEDBACK**

(71) Applicant: Creganna Unlimited Company, Ballybrit, Galway H91 VN2T (IE); TE Connectivity Solutions GmbH, 8200 Schaffhausen (CH)
(72) Inventor: McDermott, Bernard, Galway, H91 VN2T (IE); Opalinska, Marta Edyta, 8200 Schaffhausen (CH); Hart, Stephen, Galway, H91 VN2T (IE)
(74) Representative: Grünecker Patent- und Rechtsanwälte PartG mbB

(57) **Abstract**

The present invention relates to a system (1000) providing a haptic feedback improving its functionality. The system (1000) comprises a haptic feedback device (100) and communicates with a catheter (110). In operation, the haptic feedback device (100) forms a strap and each of the plurality of actuating regions (104) is arranged along a circumference a of the haptic feedback device (100). Further, in operation spatial information is transmitted from at least one of a plurality of sensing locations (112) to the haptic feedback device (100) and in accordance with the transmitted spatial information at least one of the plurality of actuating regions (104) is actuated to provide the haptic feedback.

Further, the present invention relates to a haptic feedback device (100) for providing a haptic feedback. The haptic feedback device (100) comprises a main body (102) and a plurality of actuating regions (104) that each comprise one actuator (106) and at least one ball element (108). The actuator (106) of one actuating region (104) can be actuated independently of the actuators (106) of the other actuating regions (104) to provide the haptic feedback. Further, in operation, the actuator (106) is actuated based on a signal from a catheter (110).

## Description

The present disclosure relates to a system and a haptic feedback device for providing haptic feedback.

Minimally invasive intravascular procedures allow the performance of therapeutic treatments of locations within a patient's body while requiring only relatively small access incisions. An intravascular procedure may, for example eliminate the need for open-heart surgery, reducing risks, costs, and time associated with an open-heart procedure. The intravascular procedure also enables faster recovery times with lower associated costs and risks of complication. An example of an intravascular procedure that significantly reduces procedure and recovery time and cost over conventional surgery is a heart valve replacement or repair procedure in which an artificial valve or valve repair device is guided to the heart through the patient's vasculature. For example, in a TAVR (transcatheter aortic valve replacement) procedure an aortic valve replacement device typically enters the femoral artery through a small incision in the groin area and traverses up the aorta, around the aortic arch to the location of the aortic valve where the prosthetic valve is deployed inside the native valve, taking over its function. Furthermore, the present disclosure is also applicable to various other minimally invasive procedures in which a catheter needs to be moved within a cavity, while minimizing the potential damage to boundaries of the cavity.

Interventional physicians generally use x-ray fluoroscopy for navigation and visualization of transcatheter delivery devices and procedures. This requires the physician to spend extensive hours standing in the x-ray fields with head and hands exposed to x-rays and bodies protected by heavy protective aprons. This has long-term health implications both from exposure to x-rays and orthopedic issues. Visualization of tissue using fluoroscopy is generally poor with use of enhancing contrast limited due to toxic effects on patients. Many procedures are carried out in calcified or weak and diseased tissues and there are risks that excessive device tip contact can result in damage to the vessels and can cause embolic debris to be released into bloodstream potentially causing strokes. Physicians have very little to no tactile discrimination of tissue texture, roughness, density or features at the device tip.

These problems are solved by the subject-matter of the independent claims. Advantageous examples are the subject-matter of the dependent claims.

The general idea is to provide an array of sensors on the distal end of an interventional device in an orientation around the circumference of the device with an interface device with the same spatial orientation of haptic interfaces as the orientation of sensors. This preferably allows the user to determine the location of contact of the catheter tip and the contact force at that location. Such an interface device can be a band to be placed on the wrist on the wrist or alternately on a finger, arm or other suitable area.

Such distally located tactile sensors on trans-catheter devices will assist atraumatic navigation through various anatomies. For example: can allow the determination of TAVR device tip forces against the aortic arch wall during placement, monitoring of crossing device pressure on the septum and indications of catheter tip forces to aid tracking and navigation through tortuous anatomies. Contact force feedback will allow the physician to reduce the contact force by adjusting the position of the catheter tip by rotation of the distal end, by withdrawing and advancing at a different tip angle, by reducing force or speed of advancement, by steering the tip away from contact or other means.

Such a system will also reduce reliance on fluoroscopy for navigation and enable robotic assisted procedures, thereby reducing x-ray exposure to the surgical team and allowing specialists to perform procedures remotely. Such devices will act as an extension of physicians hand inside the anatomy to allow a sense of touch and tactile discrimination of texture, roughness, density and geometries. This will facilitate the detection of specific features by internal palpation such as lumps or tumors. Such devices may also find use as training aids for inexperienced practitioners. Such navigation devices can improve efficacy and outcomes of both non-robotic and robotically assisted procedures.

In particular, the present disclosure is based on the idea to provide a haptic feedback device and further to provide a system that includes this haptic feedback device and a catheter. The haptic feedback device as described below and the system for providing the haptic feedback both individually solve the above-identified problems. The haptic feedback device provides feedback by actuating individual parts of the feedback device and the system uses an advantageous arrangement between the sensing parts at the catheter and the actuating parts at the feedback device to provide spatial information.

In particular, the present invention relates to a system for providing haptic feedback, with the system comprising a haptic feedback device and a catheter. The haptic feedback device communicates with the catheter and the haptic feedback device comprises a main body and a plurality of actuating regions. In operation, the haptic feedback device forms a strap and each of the plurality of actuating regions is arranged along a circumference of the haptic feedback device. Further, the catheter comprises a plurality of sensing locations arranged along a circumference b of a distal tip of the catheter. In operation, spatial information is transmitted from at least one of the plurality of sensing locations to the haptic feedback device and in accordance with the transmitted spatial information at least one of the plurality of actuating regions is actuated to provide the haptic feedback.

The catheter preferably is in the form of a steerable or non-steerable catheter.

According to an advantageous further development of the present disclosure, the number of actuating regions corresponds to the number of sensing locations and in operation a spatial arrangement of the plurality of actuating regions along the circumference a of the haptic feedback device corresponds to a spatial arrangement of the plurality of sensing locations along the circumference of the distal tip. The particular arrangement of the actuating regions and the sensing locations allows to transfer information detected at the sensing locations to the actuating regions and thus to provide a haptic feedback with spatial information.

According to an advantageous further development of the present disclosure, each of the plurality of actuating regions of the haptic feedback device comprises one actuator and at least one ball element and the actuator of one actuating region can be actuated independently of the actuators of the other actuating regions to provide the haptic feedback. Further, advantageously the sensing locations comprise tactile sensors, capacitive sensors or resistive sensors.

Actuating the actuators independently from each other allows a variety of information to be transmitted. Different actuators can be actuated in a certain pattern, one after the other or all at the same time and thus allowing a variety of information to be perceived. The term *"actuating"* as used can also be referred to as *"triggering"* or *"operating"* the actuators.

According to an advantageous further development of the present disclosure, the haptic feedback indicates a force that is exerted on the catheter and a magnitude of the haptic feedback can be varied in accordance with a magnitude of the force as exerted on the catheter.

According to an advantageous further development of the present disclosure, the actuating of one of the plurality of actuating regions indicates that a force is exerted on the sensing location having a spatial position that corresponds to a respective spatial position of the said actuated actuating region. With the particular advantageous arrangement, each actuation of an actuating region can easily be mapped to a corresponding sensing location. This provides an efficient way for providing detailed directional information about what part of the catheter is in contact with the cavity the catheter is inserted into.

According to an advantageous further development of the present disclosure, the haptic feedback device comprises at least four actuating regions and the catheter comprises at least four sensing locations. Providing four sensing locations and actuating regions advantageously allows to directly detect four directions - up, down, left, right - without requiring any further processing or calculating steps.

Further, the present invention also relates to a haptic feedback device for providing a haptic feedback, with the haptic feedback device comprising a main body and a plurality of actuating regions. Each of the plurality of actuating regions is arranged along a length of the haptic feedback device and each of the plurality of actuating regions comprises one actuator and at least one ball element. Further, the actuator of each actuating region can be actuated independently of the actuators of the other actuating regions to provide the haptic feedback. Further, the haptic feedback device in operation communicates with a catheter and in operation the actuator of the plurality of actuating regions is actuated based on a signal from the catheter. The possibility to actuate the actuating regions independently enables that complex and distinguishable information can be transmitted via the haptic feedback.

According to an advantageous further development of the present disclosure, the main body is formed from silicone having a first hardness and each actuating region is formed from a silicone having a second hardness, the second hardness being different from the first hardness. Fabricating the haptic feedback device from two different types of silicones mitigates the effective coupling of vibration from one actuated actuator to neighboring actuators. The different types of silicones thus isolate the actuating regions from transmitting along the main body and therefore limiting the actuation to the desired actuating region.

According to an advantageous further development of the present disclosure, the at least one ball element of each actuating region is arranged above the respective actuator and is molded into a polymer having a different material composition than the main body and the respective actuating region. The at least one ball element advantageously intensifies the vibration from the actuation region through contact with the operator and therefore allows that also small actuations can be sensed.

According to an advantageous further development of the present disclosure, each of the plurality of actuating regions comprises three ball elements. Three ball elements advantageously allow a precise yet reliably perceptible haptic feedback.

According to an advantageous further development of the present disclosure, the haptic feedback device is wearable by an operator member and in operation provides the haptic feedback to the operator based on the signal from the catheter. The haptic feedback device advantageously gives spatial information to the operator. Based on the received haptic feedback, the operator can adjust the position of the catheter. The term *"operator"* in this context refers to any person that operates and works with the haptic feedback device.

According to an advantageous further development of the present disclosure, the haptic feedback indicates a force that is exerted on the catheter and a magnitude of the haptic feedback can be varied in accordance with the magnitude of the force as exerted on the catheter. According to an advantageous further development of the present disclosure, the haptic feedback device provides a directional haptic feedback to the operator and the directional haptic feedback indicates a location of the force as exerted on the catheter. The operator feels whether and where and with what force the catheter contacts the cavity the catheter is inserted to. This allows the operator to adjust the position of the catheter or steer the catheter tip away from contact, thereby reducing the contact and tissue trauma. Further, the haptic feedback is received rapidly without any delay such that it is instantly perceived by the operator and allows him to adjust immediately.

According to an advantageous further development of the present disclosure, the haptic feedback device is connectable to a processing and storing mechanism and in operation the haptic feedback device collects and processes operational data and stores the data at the storing mechanism. Collecting the operational data is valuable for training purposes and statistics. Based on the collected data for example, future operators can be trained or a machine learning model can be developed

According to an advantageous further development of the present disclosure, the actuators of the plurality of actuating regions comprise piezoelectric actuators and/or the actuators of the plurality of actuating regions comprise eccentric rotating mass actuators and/or the actuators of the plurality of actuating regions comprise linear resonant actuators. These types of actuators provide a precise vibration and can be mass-produced. Further, the mentioned types of actuators can be encapsulated and sterilized for the use in medical devices. To better understand the present disclosure, this is explained in greater detail using the example depicted in the following Figures. Identical parts are hereby provided with identical reference numbers and identical component names. Furthermore, some features or combinations of features from the various examples shown and described may also represent independent solutions, inventive solutions or solutions according to the disclosure. In the drawings:
- **Fig. 1**: shows a schematic representation of a haptic feedback device according to an example of the present invention;
- **Fig. 2**: shows a schematic representation of the haptic feedback device according to the example of the present invention worn around the wrist of an operator;
- **Fig. 3**: shows a schematic representation of a system according to an example of the present invention;
- **Fig. 4A**: shows a schematic representation of a catheter tip according to an example of the present invention;
- **Fig. 4B**: shows a schematic representation of the catheter tip according to another example of the present invention;
- **Fig. 5**: shows a schematic representation of a main body wrist strap pre-assembly for providing haptic feedback device according to the example of the present invention, in an open configuration;
- **Fig. 6**: shows a schematic representation of molds that can be used to fabricate the haptic feedback device of the present invention;
- **Fig. 7**: shows a schematic representation of the arrangement of the actuating regions at the haptic feedback device according to one example of the present invention;
- **Fig. 8**: shows a schematic representation of another arrangement of the actuating regions at the haptic feedback device according to another example of the present invention;
- **Fig. 9**: shows a schematic representation of the sensing directions of actuation according to one example of the present invention.

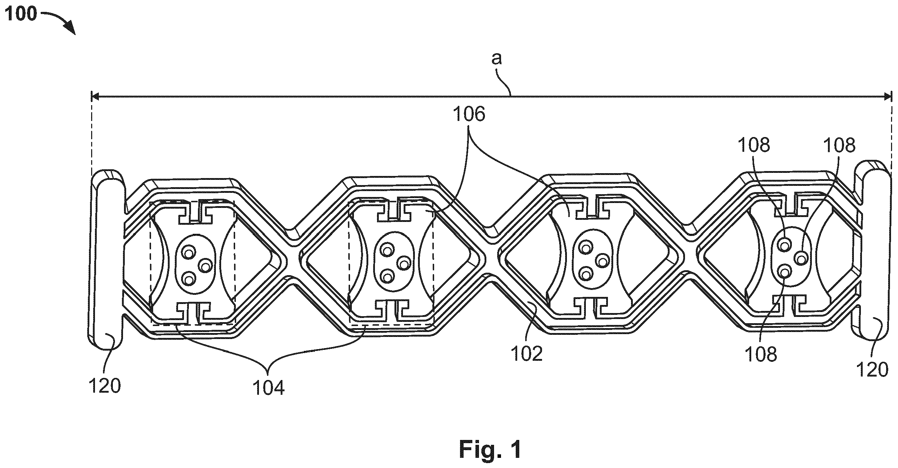

Fig. 1 shows an example of a haptic feedback device 100 according to the present invention. The haptic feedback device 100 comprises a main body 102 and a plurality of actuating regions 104. In Fig. 1, the haptic feedback device 100 is exemplarily shown with four actuating regions 104.

The plurality of actuating regions 104 are arranged along a length a of the haptic feedback device 100. It is clear that also more than four actuating regions 104 can be positioned along the length a. The number of actuating regions 104 can be varied depending on the application and the required level of complexity of the haptic feedback. In Fig. 1, the plurality of actuating regions 104 are exemplarily arranged equidistantly along the length a, but they may also be arranged in a different order.

Each actuating region 104 comprises one actuator 106 and at least one ball element 108. Here exemplarily each actuating region 104 comprises three ball elements 108. The ball elements 108 are arranged above the actuating region 104.

The main body 102 is formed from a silicone or a similar material having a first hardness and each actuating region 104 is formed from a silicone or a similar material having a second hardness. The first hardness is different from the first hardness. The hardness of silicone as a type of polymer is defined in Shore. Here, the first hardness and the second hardness refer to different Shore values. The main body 102 is fabricated from a material having a higher Shore value than the material from which the actuating region 104 is fabricated. A material having a higher Shore value is more resistant to indentation and thus harder. A material with a lower Shore value is softer. Thus, the main body 102 is preferably formed from a harder, more rigid silicone. The actuating regions 104 are preferably formed from a silicone having a lower value of Shore and thus from a softer silicone. In this context a lower Shore value is around 10 Shore A and a higher Shore value is around 30 Shore A.

Thereby, the particular design of the haptic feedback device 100 having a main body 102 that is fabricated from a different silicone than the actuating region 104 has the advantage that two neighboring actuators are isolated from each other. This minimizes adjacent vibrations such that the vibration of one actuator does not spread to neighboring actuators. Further, air gaps can be provided within the silicone structure to further improve the isolation between neighboring actuators. Further, the haptic feedback device may also be formed from other materials than silicone or polymer, as for example from fabric.

Further, in operation each actuator 106 of the plurality of actuating regions 104 can be actuated independently of the actuators 106 of the other actuating regions 104 to provide a haptic feedback.

In operation, the haptic feedback device 100 communicates with a catheter 110. The catheter 110 according to this invention can be in the form of a steerable or non-steerable catheter.

During the use of the haptic feedback device 100, the catheter 110 provides a signal upon which the actuating regions 104 are actuated. The communication between the catheter 110 and the haptic feedback device can be in the form of a wireless communication or a communication via a cable. Further, the haptic feedback device 100 may be connected to other devices that used during the procedure.

The haptic feedback device 100 then gives a haptic feedback to indicate a state or change in the state of the catheter 110. The haptic feedback device 100 is worn by an operator 118 and exemplarily can be worn around the wrist, arm, or other suitable body part. Therefore, when worn by an operator, the length a of the haptic feedback device 100 corresponds to the circumference a of the haptic feedback device 10. The haptic feedback device 100 further also includes magnets 120 to allow a fast and flexible fastening at the desired body part of the operator 118. Further, it allows to keep a constant relative position between the actuators.

Further, by being able to actuate the actuators 106 of the plurality of actuating regions 104 independently of each other, the haptic feedback device 100 advantageously provides directional haptic feedback to the operator 118.

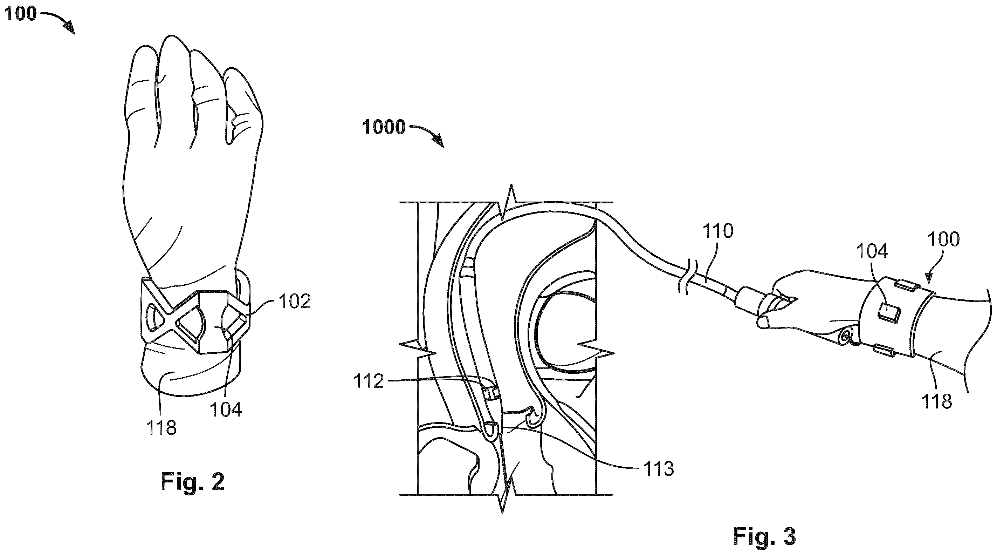

**Fig. 2** shows an exemplarily use of the haptic feedback device 100 around the wrist of the operator 118. The haptic feedback device 100 can be in the form of a braided wristband 102 that is fastened by the magnets (see Fig. 1). The arrangement of the actuating regions 104 along the length of the haptic feedback device 100 can be varied and adapted based on the positions where a haptic feedback should be provided to the operator 118. The actuators are actuated based on the signal and the actuation transfers to the ball elements 108, which pass the actuation to the operator 118.

The ball elements 108 that are part of every actuating region 104 are not visible in Fig. 2. The ball elements 108 are arranged on the side that faces the operator 118 and thus in operation contact the operator 118. Below the ball elements 108 and thus circumferentially to the outside, the actuator of the actuating region 104 is positioned.

The ball elements 108 are preferably formed from metal and are used to improve the skin coupling of the actuation as actuated by the actuator. The ball elements 108 preferably intensify the vibration sensation. Thus, the operator 118 is able to sense even small vibrations from the actuators. Further, by using one or more than one connecting points between the haptic interface 110 and the operator 118 the actuation can be precisely perceived.

Depending on the received signal, either one or more than one actuator 104 is actuated such that the operator senses an actuation transmitted via the ball elements 108 either on one or on multiple positions.

The ball elements 108 are preferably molded into a polymer that has a material composition that is different from the material composition of the main body 102 and the actuating regions 104. The ball element 108 is preferably molded into a polymer that allows transmitting a signal wave actuated from the actuator 106 to the ball element 108.

Types of actuators that are preferably used are either piezoelectric actuators or eccentric rotating mass actuators or linear resonant actuators.

**Fig. 3** shows a very simplified version of a system 1000 with the haptic feedback device 100 that communicates with a catheter 110. The communication can be in the form of a connection via a cable or wireless.

In the shown example, the haptic feedback device 100 is positioned around the wrist of the operator 118. As can be seen, the position of the actuating regions 104 is shown in an exaggerated way by protruding outwardly from the haptic feedback device 100. The protruding regions only indicate where the actuating regions 104 can be positioned with respect to the circumference of the main body 102 of the haptic feedback device 100.

The catheter 110 includes a distal tip 113 that is positioned at the distal end that is the end of the catheter device furthest from the feedback device. The catheter 110 may be inserted into a bodily lumen; blood vessel, pulmonary system, digestive system or other body cavities.

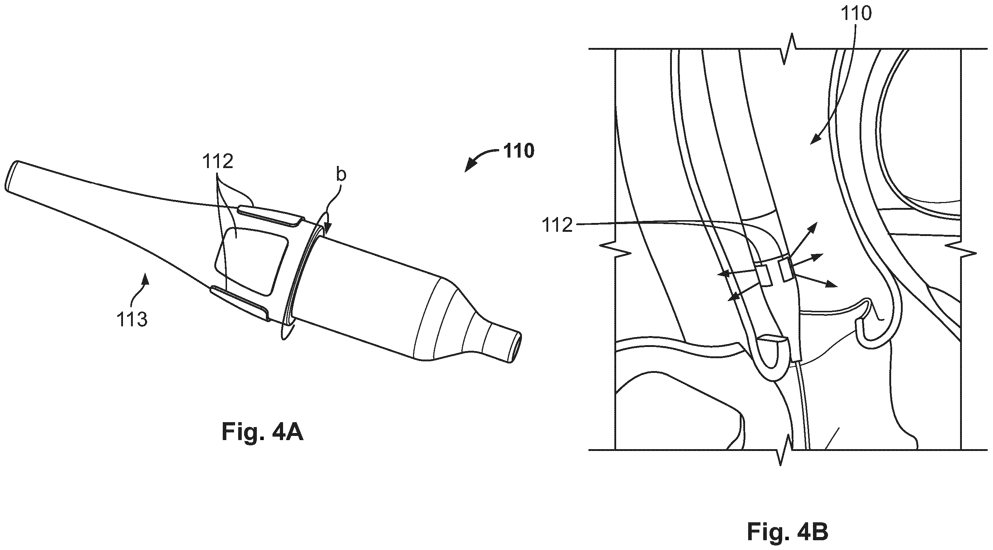

**Fig. 4A** shows an example of the distal tip 113 of the catheter 110, which is connectable to the haptic feedback device 100 with the features as mentioned above. The catheter 110 comprises a plurality of sensing locations 112 arranged along a circumference b of the catheter tip 113. The sensing locations comprise tactile sensors, capacitive sensors, resistive sensors or other types of sensors.

In operation, the catheter 110 is inserted into a cavity such as a blood vessel and advanced until the desired location where the medical procedure is to be conducted is reached. When advancing the catheter forward through the cavity, the sensing locations 112 sense whenever the catheter tip 113 touches a wall of the cavity and give a feedback signal to the haptic feedback device 100. This feedback allows the operator to reduce the contact force by adjusting the position of the catheter tip, by rotation of the distal end, by withdrawing and advancing at a different tip angle, by reducing force or speed of advancement and/or by steering the tip away from contact or other means.

When the haptic feedback device 100 is worn it forms a strap around for example the wrist of the operator 118. Thus, the plurality of actuating regions 104 extend along the circumference a of the haptic feedback device 100. In other examples, the haptic feedback device can form a strap around a finger, arm, head or any other suitable area.

During the use, the sensing locations 112 transmit spatial information to the haptic feedback device 100 and based on the transmitted information one of the actuators 106 is actuated. The spatial information may relate to a directional information including a location, position or orientation of the catheter tip 113.

The sensing locations may constantly transmit a signal and the haptic feedback device or another device in communication with the catheter and the sensing locations may determine based on the received signal whether a haptic feedback should be provided or not.

Another possibility is that the sensing locations only transmit a signal when they have been activated and in accordance with the transmitted information, at least one of the actuating regions 104 is actuated and provides the haptic feedback. A sensing location 112 can be activated in different ways depending on the type of sensing location. The sensing location may be a force sensor that is activated as soon as a force is exerted on the sensing location. The sensing location can also be an ultrasonic transducer that measures a distance between the sensing location and a wall by Time-of-Flight measurement. Such a sensing location may be activated and transmits a signal when a certain distance threshold is undercut. Other types of sensing locations that comprise sensors, which transmit different information such as a temperature, conductivity or other sensing parameters may be used.

In one example, the haptic feedback that is perceived by the operator 118 indicates a force that is exerted on the catheter 110 and in particular is exerted on one or on multiple sensing locations 112. Thereby, depending on a magnitude of force that is exerted on one or multiple sensing locations 112, the magnitude of haptic feedback can be varied. Thus, the operator 118 can easily feel the amount of contact between the catheter tip 113 and the cavity the catheter 110 is inserted to. It is clear that

Further, the vibration level of the actuators can not only be adjusted based on the force that is exerted on the corresponding sensing location, but may also be adjusted based on the sensitivity of the operator or based on general settings. The vibration frequency of the actuators exemplarily are between 125 Hz - 300 Hz. Further, the vibration of the actuators can be coded meaning that the vibration is given in the form of a pattern. The operator can identify the pattern as symbols or words, which widens the amount and complexity of information that can be transmitted and derived. The coding of the vibration can be realized using techniques such as pulse width modulation or pulse sequences.

The perceived information from the sensing locations 112 may also be transmitted to further devices beside of the haptic feedback device 100. Exemplarily, the information is also transmitted to a display device as numerical or directional information. At the display device, various information such as the force levels or a directional vector can be shown. The vector having a magnitude and angle indicates how to best steer the catheter tip for least contact force. In this way, the operator not only receives a haptic feedback but also a visual feedback, which helps to further reduce the possibility of excessive contact of the walls of the cavity.

In a preferred example, the number of actuating regions 104 corresponds to the number of sensing locations 112. Meaning that for each sensing location 112 a corresponding actuating region 104 is positioned at the haptic feedback device 100. Thereby, when the haptic feedback device 100 is closed and forms a strap, the spatial arrangement of the actuating regions 104 along the circumference a of the haptic feedback device 100 matches the spatial arrangement of the sensing locations 112 along the circumference b of the distal tip 113.

Thus, in this example for each actuating region 104 at the haptic feedback device 100 a corresponding sensing location 112 having the same relative position along the circumference is provided at the catheter 110. In another example, the catheter 110 comprises more sensing locations 112 at the catheter tip 113 than the haptic feedback device 100 includes actuating regions 104. In such an example, the signal from multiple sensing locations is mapped to one actuator. Thus, each actuator receives its signal from a group of sensing locations. This is an advantageous setup for systems used in very small and complex cavities where many sensing locations are required to correctly provide a haptic feedback.

Further, the haptic interface is not only used to give the operator a haptic feedback about the magnitude of contact but may also used for indicating the location of contact. As mentioned above, the actuators 106 that are part of the haptic feedback device 100 can be actuated independently of each other. Meaning that an actuator 106 of a first actuating region 104 can be actuated independently of the actuators 106 of the other actuating regions 104. This gives the operator 118 a directional feedback. The directional feedback may give the operator 118 information about the location of contact and thus about which sensing location 112 contacted the cavity. The location of contact in this contact refers to information about the position and orientation of contact.

The actuation of one actuator may indicate that a force is exerted on the sensing location 112 that has the same spatial position that corresponds to the respective spatial position of the actuated actuator. Thereby the type of information that is transmitted to the operator via the directional feedback may be different for different procedures and/or different catheter types with different sensing locations.

As explained above, the haptic feedback may give the operator information about a magnitude of contact force of the catheter tip 113 and/or a location of the contact of the catheter tip. The haptic feedback may also be used to nearly prevent a contact of the catheter tip with the cavity into which the catheter is inserted. The catheter tip 113 in this case includes sensing locations 112 that comprise ultrasonic transducers via which a distance of the sensing locations 112 to the walls of the cavity can be measured. **Fig. 4B** shows exemplarily a catheter 110 with a catheter tip 113 having sensing locations 112 positioned along a circumference of the catheter tip 113 that is inserted into a cavity.

Here, the sensing locations are in the form of ultrasonic transducers, which measure the distance to the wall of the cavity by Time-of-Flight measurement. This shows that the exact design of the catheter tip 113 and the exact position of the sensing locations 112 along a length of the catheter can be varied and still provide the advantageous haptic feedback when communicating with the haptic feedback device. The haptic feedback device 100 then gives the operator 118 a haptic feedback as soon as a predetermined distance from the catheter tip 113 to the walls of the cavity is undercut. Thus, an actuation of an actuator indicates that the catheter tip is about to touch the wall of the cavity and indicates the operator 118 that the position should be adjusted.

Further, it is clear that the haptic feedback can be used to indicate a variety of information. The actuators can be actuated independently of each other or in combination and can therefore be used to transmit a variety of information to the operator 118.

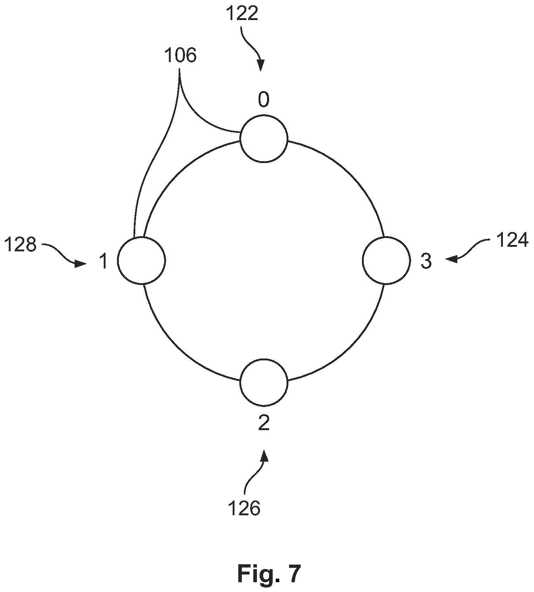

It can be also advantageous to map the signal from one sensing location 112 to an actuator having a different spatial position. As can be seen in **Figures 7 - 9****,** the haptic feedback device 100 exemplarily comprises four or more actuating regions 104 and the catheter tip 113 here also comprises four or more sensing locations 112. The four actuating regions 104 as shown in Fig. 7 are numbered from 0 - 3 and accordingly the sensing locations 112 having the same relative position can be numbered.

As described above, as soon as sensing location 0 is activated a signal is provided to the haptic feedback device 100. This signal may be perceived by actuating region 0 and indicates that a force is exerted on sensing location 0. However, a signal from sensing location 0 may also be mapped to an actuating region having the opposite relative position, such as in this example actuating region 2. The same applies for sensing locations 1 and 3. In case one of these sensing locations transmits a signal, the opposite actuating region may be activated. The setup which sensing location is mapped to which actuating region can be flexibly configured.

The method to provide a haptic feedback at the haptic feedback device 100 can be described as the following. Sensing with at least one of the sensing locations 112 arranged at the catheter tip 113 of the catheter 110 and providing a corresponding signal to the haptic feedback device 100. The haptic feedback device 100 then in accordance with the received signal actuates one or more actuating regions 104.

Based on the at least one triggered actuator in the actuating region 104, the operator 118 senses the actuation and can identify the actuated direction. For example, the four actuating regions 104 in Fig. 7 can be identified as providing an actuation in one of the directions 122- 128. For example, if the actuator 0 is actuated, the operator may identify that the actuation comes from a first direction 122. The operator accordingly may identify an actuation in the second direction 124, the third direction 126 or the fourth direction 128.

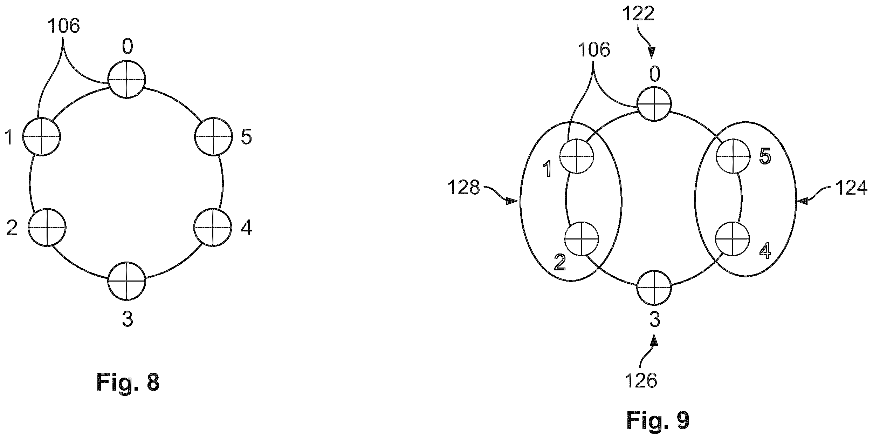

**Fig. 8 and 9** show a schematic representation of a haptic interface having six actuating regions and thus six actuators 106 placed equidistantly along the circumference. The actuators are numbered from 0 to 5 and can be actuated independently of each other. When the six actuators are actuated, each individual actuator can either be identified individually (Fig, 8) or based on the actuation of an actuator a direction can be identified (Fig. 9).

All mentioned features and steps as described above are also applicable for a haptic feedback device 100 having six actuating regions 104 and are of course also applicable for a different number of actuating regions 104.

Fig. 8 shows that an actuation of one of the six actuators may be exactly identified as coming from this actuator. Fig. 9 shows an example where the actuation of one of the six actuators is mapped to an actuation from one of the four directions 122 - 128.

An actuation of one or both of the actuators 1 and 2 is identified as an actuation coming from the fourth direction 128, and an actuation of one of the actuators 4 and 5 is identified as an actuation coming from the second direction 124. The actuation of the actuator 0 is identified as coming from the first direction 122 and the actuation of the actuator 3 is identified as coming from the third direction 126.

Further, during the use of the haptic feedback device 100 data that includes operational information may be recorded and stored in a computational processing and storing mechanism that is connected to the haptic feedback device 100. The data may include information about the number of contacts, the magnitude of contacts, the location of the contact etc. The recorded data from a plurality of procedures can then be collected in a large database and clustered there. Further information that is also stored in the database is the outcome of the procedure and in particular the medical condition of the patient that has undergone the procedure. Having this large database and the contact of the catheter tip and the condition of the patient, a machine learning algorithm can be trained to find a correlation between all the data with respect to clinical outcomes post surgery.

The machine learning algorithm may also be used for supervised learning, meaning that the collected contact data is already labeled and correlated to a condition of the patient. The supervised machine learning is then used to train operators and give them feedback about the impact of a contact. Whenever an operator receives haptic feedback, the machine learning algorithm can predict the potential outcome and condition of the patient and give an indication about the impact of the contact.

This advantageously improves the knowledge an operator has about whether additional effects or diseases may be caused by the procedure and whether additional steps need to be taken. The haptic feedback and in particular storing the data allows an operator to give a much better prediction and conclusion about the course of the procedure. The operator not only has information about whether and how the catheter contacted the cavity but also can compare the contact and the force to previous procedures. This advantageously reduces the probability of unforeseen repercussions.

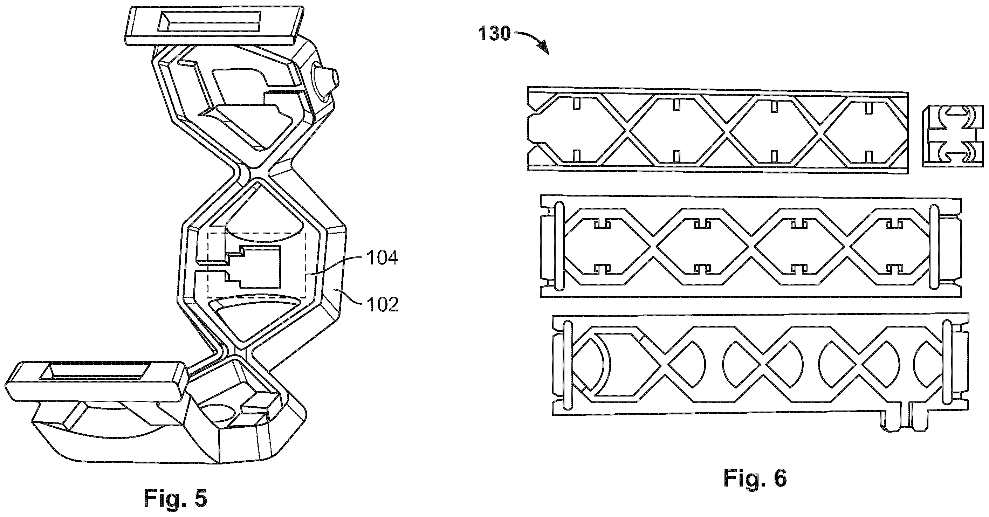

All parts of the haptic feedback device 100 such as the main body 102 and the actuating region 104 can be fabricated using a 3D printing method. Thereby, also the actuating region 104 with the polymer structure into which the ball element 108 is molded can be 3D printed. **Fig. 5** shows a pre-assembled version of the haptic feedback device 100 in an open configuration, without an actuator, ball element and the polymer as part of the actuating region 104. Further, **Fig. 6** shows molds 130 that can also be used to fabricate the main body 102 and the actuating region 104 of the haptic feedback interface 100.

Further, as already mentioned above, the features described herein with respect to the haptic feedback device are of course also applicable for the described system comprising the catheter and the haptic feedback device and vice versa.

**List of reference signs**

| **Reference Numeral** | **Description** |
|---|---|
| 100 | haptic feedback device |
| 102 | main body |
| 104 | actuating region |
| 106 | actuator |
| 108 | ball element |
| 110 | catheter |
| 112 | sensing location |
| 113 | distal tip |
| 118 | operator |
| 120 | magnets |
| 122 | first direction |
| 124 | second direction |
| 126 | third direction |
| 128 | fourth direction |
| 130 | 3D printed molds |
| 1000 | haptic feedback system |
| a | length and circumference of the haptic feedback device |
| b | circumference of the distal tip |

## Claims

1. System (1000) for providing haptic feedback, the system (1000) comprising a catheter (110) and a haptic feedback device (100);
wherein the catheter (110) communicates with the haptic feedback device (100);
wherein of the haptic feedback device (100) comprises a main body (102) and a plurality of actuating regions (104);
wherein, in operation, the haptic feedback device (100) forms a strap and each of the plurality of actuating regions (104) is arranged along a circumference (a) of the haptic feedback device (100); and
wherein the catheter (110) comprises a plurality of sensing locations (112) arranged along a circumference (b) of a distal tip (113) of the catheter (110);
wherein, in operation, spatial information is transmitted from at least one of the plurality of sensing locations (112) to the haptic feedback device (100) and
wherein in accordance with the transmitted spatial information at least one of the plurality of actuating regions (104) is actuated to provide the haptic feedback.

2. The system (1000) according to claim 1,
wherein the number of actuating regions (104) corresponds to the number of sensing locations (112); and
wherein, in operation, a spatial arrangement of the plurality of actuating regions (104) along the circumference (a) of the haptic feedback device (100) corresponds to a spatial arrangement of the plurality of sensing locations (112) along the circumference (b) of the distal tip (113).

3. The system (1000) according to one of the preceding claims,
wherein the sensing locations (112) comprise tactile sensors, capacitive sensors or resistive sensors;
wherein each of the plurality of actuating regions (104) of the haptic feedback device (100) comprises one actuator (106) and at least one ball element (108);
wherein the actuator (106) of one actuating region (104) can be actuated independently of the actuators (106) of the other actuating regions (104) to provide the haptic feedback.

4. The system (1000) according to one of the preceding claims,
wherein the haptic feedback indicates a force that is exerted on the catheter (110) and
wherein a magnitude of the haptic feedback can be varied in accordance with a magnitude of the force as exerted on the catheter (110).

5. The system (1000) according to claim 2,
wherein the actuation of one of the plurality of actuating regions (104) indicates that a force is exerted on the sensing location (112) having a spatial position that corresponds to a respective spatial position of the said actuated actuating region (104).

6. The system (1000) according to one of the preceding claims, wherein the haptic feedback device (100) comprises at least four actuating regions (104) and wherein the catheter (110) comprises at least four sensing locations (112).

7. Haptic feedback device (100) for providing a haptic feedback, the haptic feedback device (100) comprising:
a main body (102) and a plurality of actuating regions (104);
wherein each of the plurality of actuating regions is arranged along a length (a) of the haptic feedback device (100); and
wherein each of the plurality of actuating regions (104) comprises one actuator (106) and at least one ball element (108);
wherein the actuator (106) of one actuating region (104) can be actuated independently of the actuators (106) of the other actuating regions (104) to provide the haptic feedback;
wherein, in operation, the haptic feedback device (100) communicates with a catheter (110) and
wherein, in operation, the actuator (106) of the plurality of actuating regions (104) is actuated based on a signal from the catheter (110).

8. The haptic feedback device (100) according to claim 7, wherein
the main body (102) is formed from a silicone having a first hardness and each actuating region (104) is formed from a silicone having a second hardness, the second hardness being different from the first hardness.

9. The haptic feedback device (100) according to one of claims 7 or 8, wherein
the at least one ball element (108) of each actuating region (104) is arranged above the respective actuator (106) and is molded into a polymer having a different material composition than the main body (102) and the respective actuating region (104).

10. The haptic feedback device (100) according to one of claims 7 to 9, wherein
each of the plurality of actuating regions (104) comprises three ball elements (108).

11. The haptic feedback device (100) according to one of claims 7 to 10, wherein the haptic feedback device (100) is wearable by an operator (118) and in operation provides the haptic feedback to the operator (118) based on the signal from the catheter (110).

12. The haptic feedback device (100) according to claim 11, wherein the haptic feedback indicates a force that is exerted on the catheter (110) and
wherein a magnitude of the haptic feedback can be varied in accordance with the magnitude of the force as exerted on the catheter (110).

13. The haptic feedback device (100) according to one of claims 11 or 12, wherein the haptic feedback device (100) provides a directional haptic feedback to the operator (118) and wherein the directional haptic feedback indicates a location of the force as exerted on the catheter (110).

14. The haptic feedback device (100) according to one claims 7 to 13, wherein the haptic feedback device (100) is connectable to a processing and storing mechanism,
wherein, in operation, the haptic feedback device (100) collects and processes operational data and stores the data at the storing mechanism.

15. The haptic feedback device (100) according to one of claims 7 to 14,
wherein the actuators (106) of the plurality of actuating regions (104) comprise piezoelectric actuators and/or
wherein the actuators (106) of the plurality of actuating regions (104) comprise eccentric rotating mass actuators and/or
wherein the actuators (106) of the plurality of actuating regions (104) comprise linear resonant actuators.
